# EUROPEAN PATENT SPECIFICATION

(11) **EP 1 888 099 B1**
(45) Date of publication and mention of the grant of the patent: **26.03.2014**
(21) Application number: 06758909.3
(22) Date of filing: 04.05.2006
(51) Int. Cl.: A61K 35/36, A61K 38/17, A61L 15/32, A61L 31/16

(54) **Hydratable keratin compositions**
Hydrierbare Keratin-Zusammensetzungen
Compositions hydratables a base de kératine

(30) Priority: 06.05.2005 US 678859 P
(43) Date of publication of application: 20.02.2008
(73) Proprietor: Keraplast Technologies Ltd., San Antonio, TX 78258 (US)
(72) Inventor: WU, Chunyong, Plymouth, MN 55446 (US); LI, Jun, Cambridge, MA 02139 (US); WICKS, Douglas, Hattiesburg, Mississippi 39401 (US); MORGAN, Sarah School of Polymers and High Performance Materials, Hattiesburg, MS 39406-0001 (US); SMITH, Robert, Jackson, Mississippi 39211 (US)
(74) Representative: Jones, Nicholas Andrew
(86) International application number: PCT/US2006/016779
(87) International publication number: WO 2006/121700

(56) References cited:
- WO-A-03/006531
- JP-A- 2006 239 093
- US-A- 3 971 159
- US-A- 5 660 857
- US-A1- 2003 035 820
- US-B1- 6 270 793
- US-B1- 6 316 598
- US-B1- 6 649 740
- GÜTT: "Collagen - A biomaterial for the future" KOSMETIK INTERNATIONAL OFFPRINT 4/2003, 2003, pages 1-2, XP002511675 Retrieved from the Internet: URL:www.skin-healthcare.de/en/aktuelles/do wnloads/Ki-Druck-engl.pdf> [retrieved on 2009-01-22]

## Description

### Background of the Invention

Keratins, as the major structural proteins of all epithelial cells, play important roles for wound healing and tissue recovery Proper wound healing processes involve the covering of the wound by keratinocytes, cells which manufacture keratins and migrate from the wound edge Keratins have been found to be necessary for reepitthelization during wound healing (Coulombe, P.A., Prog In Dermatology, 2003, 37(1), 219-230; Bruen, et al., J. Surg. Res 2004, 120(1), 12 20, McGowan and Coulombe, J Cell Biol. 1998, 143 (2), 469-486; Paladini, et al., J Cell Biol. 1996, 132(3), 381-97) In addition, keratin based biomaterials are capable of protecting injured tissue and maintaining a moist environment for promotion of wound healing. Certain keratin biomaterials are also effective for delivery of growth factors and pharmaceutical agents to promote wound healing for acute and chronic wounds (US. Patent Nos 5,932,552, 5,948,432, and 6 124.265). US 6,270,793 describes a hydratable, highly absorbent keratin solid fiber or powder, preferably prepared from hair which, following chemical treatment, retains a proportion of water soluble peptide chains, the solid fiber or powder being dried by evaporation under vacuum. US2003/0035820 discloses soluble keratin which can be cast into various shapes

### Summary

The present disclosure includes hydratable absorbents and films fabricated with simple, repeatable processes from hair. Freeze drying produces a material with instantaneous water absorption to over 20 times its weight in water Films produced are flexible and uniform with water absorption 7 - 8 times the original weight.

The present disclosure may be described therefore as a composition comprising an absorbent keratin material obtained from a hair filament, wherein the absorbent material is substantially free ot water soluble keratin proteins and peptides and wherein the filament is produced by saturating the filament with aqueous solvent to obtain an expanded filament and subsequently removing the water by sublimation to obtain an absorbent material. The described composition has been shown to absorb at least 20 times its weight of water. Sublimation may be accomplished by any means known m the art and is preferably accomplished by a freeze-drying technique. Compositions as described herein may be made from various keratin sources, and preferably are made or obtained from hair of a human or animal, including wool.

The disclosed compositions may be described in certain embodiments as compositions made by the process comprising:
oxidizing hair or wool effective to disrupt a major portion of the cystine disulfide bonds in the hair or wool;
removing substantially all the proteins and peptides that are water soluble at neutral pH from the composition;
swelling the composition by saturation with aqueous solution; and
freeze-drying the composition.

### Brief Description of the Drawing

The following drawings form part of the present specification and are included to further demonstrate certain aspects of the present invention. The invention may be better understood by reference to one or more of these drawings in combination with the detailed description of specific embodiments presented herein.
Figure IA is a light micrograph of a human hair after vacuum drying (comparative).
Figure IB is a light micrograph of a human hair after freeze-drying (of the invention).

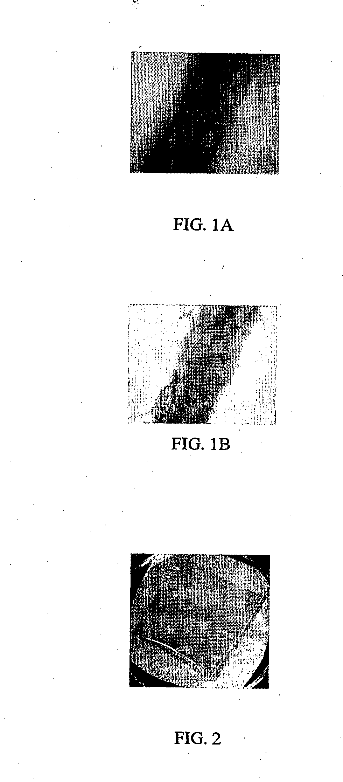

Figure 2 is a photograph of a sheet material formed of the soluble portion of hair.

### Detailed Description

Oxidation of Human Hair. Sixty grams of human hair was washed with 10% Vera® detergent from Fisher Scientific, and then rinsed with copious amounts of deionized water. The water was removed by drying overnight at ambient conditions. The resultant clean hair was added to 1000 ml 2% peracetic acid, boiled for 2 hours, and filtered. The collected hair was further rinsed in 100 ml ethanol, filtered, and dried under vacuum at room temperature overnight.

Titration of Oxidized Human Hair. Dry, oxidized hair prepared as described in the previous paragraph was mixed with deionized water, and then titrated with 0.1M sodium hydroxide to pH 7. The neutralized hair slurry was filtered and washed with copious amounts of deionized water until a light-yellowish hydrogel formed in the filtration funnel. Both the extract and the hydrogel were collected to make the corresponding products.

Making keratin absorbent. The hydrogel was freeze-dried or alternatively, vacuum dried to remove the entrapped water. The hydrogel made from freeze-drying maintained the texture of the swollen hydrogel, and formed a white, sponge-like absorbent. The absorbent made from vacuum drying was hard and brittle.

Making keratin film. The extract was titrated with HCl to obtain a precipitate. The precipitate was filtered, rinsed with copious amounts of pure ethanol, and vacuum dried. The resultant keratin was a light-gray powder. The keratin powder was further dissolved to make a 10% aqueous solution by addition of ammonia to a pH of no more than 8, and then glycerin was added in amount to achieve 30% glycerin relative to . protein. The 10ml solution was added to a Petri dish (3.5" diameter), and was dried overnight at room temperature to form a flexible film.

Water absorption testing. Water absorption for the keratin absorbent and film is tested by drop-wise addition of water until excess water is observed that is not absorbed by the keratin hydrogel or the swollen film, respectively.

Hair keratin is in the form of microfibrils and an amorphous "glue" matrix beneath the tubular outer cuticle of human hair, and is rich in sulfur content. Its integrity is mainly due to the presence of disulfide linkages through cystine units. To make a keratin hydrogel, the disulfide linkage is ruptured, preferably by partial oxidation, to form hydrophilic pendant groups primarily on the cysteine amino acid side chains. These groups are primarily sulfonic acid groups, which can complex with water. In preferred embodiments, 2% peracetic acid or the equivalent is used as the oxidation agent to partially convert disulfide linkages into pendant sulfonic acid groups. A sulfonic acid group as used herein may be defined as a sulfur atom bonded to one, two, or three oxygen atoms. The disclosed materials are not limited to oxidized keratin, as the disulfide bonds may also be broken by reductive techniques that do not result in formation of sulfonic acid goups.

After oxidation, a fraction of oxidized keratin is insoluble in aqueous solution at neutral pH. This fraction of the keratin absorbs water and forms a hydrogel upon contact with water. It is an aspect of the present disclosure that the method of drying the insoluble fraction of the keratin affects the structure and function of the insoluble material. For example, removing the residual water from the structure by sublimation, as in freeze-drying, for example, results in an expanded, highly absorbent material. This structure is shown in Figure 1B and is shown to absorb over 20 times its weight of water to form a hydrogel. Because the filament maintains at least partial structural integrity and is highly absorbent, this material is an excellent biomaterial for use in cell scaffolding applications and for the delivery of active agents in wound healing applications, for example.

The soluble portion of oxidized hair that is separated from the insoluble absorbent includes alpha keratin from the hair cortex. The oxidized hair is easily dissolved to form keratin solutions with controlled concentrations. Further, by the addition of a biocompatible plasticizer, glycerin for example, the solution can be cast into a thin flexible film. As is shown in Table 1, the resultant film was clear and uniform, and was able to absorb 7∼8 times its weight in water without losing its integrity. Considering the incorporation of glycerin into the film, this is excellent absorption capability. The integrity of the films can be further improved with increased crosslinking.

**Table 1. Keratin film thickness, yellow index and swelling ratio**

| Sample | 1 | 2 | 3 | 4 | 5 |
|---|---|---|---|---|---|
| Thickness (µm) | 87.3±15.0 | 83.7±14.6 | 87.7±15.5 | 80.5±12.5 | 86.7±21.3 |
| Yellow index | 51.7±4.4 | 52.0±2:3 | 53.0±3.9 | 53.0±2.5 | 53.2±3.8 |
| Swelling ratio | 7.9 | 8.81 | 7.62 | --- | 7.86 |

Preferred uses of the described absorbent and films are as components in a wound dressing or, especially in the case of the absorbent as a tissue engineering cell scaffold for implant applications or for the delivery of pharmaceutically active agents topically, or to a wound or from the surface of an implant, for example. Other uses would include as a controlled or sustained release drug delivery material. Examples of materials that may be included in the absorbent include, but are not limited to anti-bacterial or antibiotic agents, ointments, or biologics such as growth factors or cytokines, collagens, or glucosaminoglycans. Yet another preferred use is in skin care products. The absorbent material is preferably obtained from hair filament and but any source of insoluble or beta-keratin may be used, such as hair, wool, or fur of any mammal as well as claws, hooves, or nails, or even avian products such as feathers or hard keratin materials. In most preferred embodiments, the keratin is obtained from hair or wool including human hair or wool, hair or fur from domestic or agricultural animals. In certain embodiments, it may be advantageous to obtain such products from the hair of a subject who will be subsequently treated with the keratin-derived product.

The materials described herein may be produced in various carrier formulations and in various shapes and dimensions including powders, sheets, gels, ointments, lotions, sprays, tablet, lozenge, or the material may be molded into three dimensional objects such as sponges, screws, or in any shape suitable for an implant, including cosmetic implants, or as a tissue expanding material. The disclosed materials may be formed into useful objects such as sheets. bandages, implants, bone screws, spinal cages, etc. either as pure keratin materials or combined with polymeric binders, thickeners, crosslinkers, structural molecules, minerals, ceramics, metals, metal alloys, silicone, and other biocompatible materials.

The porous materials described herein are also useful as cell growth or tissue engineering scaffolds and support the growth of many cell or tissue types For example, the materials may support the growth of keratinocytes, fibroblasts, endothelial cells, osteoblasts, chondrocytes or hepatocytes, either in vitro or in vivo in an implant, or bonded to the surface of an implant. Any type of implant mat be coated with the keratin obtained products described herein in order to reduce the inflammatory response to the implant, or to deliver a pharmaceutical or biological agent from the implant. Such implants would include, but are not limited to pacemakers, stents, orthopedic implants, urological implants, dental implants, breast implants, and maxillofacial implants.

The described materials are also useful in applications in which absorption of liquid is needed, such as in diapers, feminine hygiene products, bandages, for example, or in applications including environmental cleanup, soil amendment, desert reclamation or other areas where there is a need to absorb or maintain large amounts of water or liquid.

All of the compositions and methods disclosed and claimed herein can be made and executed without undue experimentation in light of the present disclosure.

## Claims

1. A composition comprising an absorbent keratin material obtained from a hair filament, wherein the absorbent material is substantially free of water soluble keratin proteins and peptides and wherein the filament is produced by saturating the filament with aqueous solvent to obtain an expanded filament and subsequently removing the water by sublimation to obtain an absorbent material.

2. The composition of claim 1 , wherein the absorbent material absorbs at least 20 times its weight of water.

3. The composition of claim 1 , wherein sublimation is accomplished by freeze-drying the absorbent keratin material.

4. The composition of claim 1 , wherein the hair filament is a human hair filament.

5. The composition of claim 1 , wherein the hair filament is an animal hair or wool filament.

6. A composition made by the process comprising: oxidizing hair or wool effective to disrupt a major portion of the cystine disulfide bonds in the hair or wool; removing substantially all the proteins and peptides that are water soluble at neutral pH from the composition; swelling the composition by saturation with aqueous solution; and freeze-drying the composition.

7. Use of a composition according to any preceding claim in the preparation of a water absorbent film.

8. Use of a composition according to any of claims 1 to 6 in the preparation of a wound dressing, a cell growth scaffold, a controlled or sustained release drug delivery material, a skin care product, a water absorbent bandage, a diaper, an absorbent feminine hygiene product or an environmental liquid absorbent.

## Patentansprüche

1. Eine Zusammensetzung die ein absorbierendes Keratin-Material, das aus einem Haar-Filament gewonnen ist, aufweist, wobei das absorbierende Material im Wesentlichen frei von wasserlöslichen Keratin-Proteinen und -peptiden ist und wobei das Filament durch Sättigen des Filaments mit einer wässrigen Lösung produziert wird, um ein expandiertes Filament zu gewinnen und anschließend das Wasser durch Sublimation zu entfernen, um ein absorbierendes Material zu gewinnen.

2. Zusammensetzung nach Anspruch 1, wobei das absorbierende Material wenigstens das 20-fache seines Gewichts an Wasser absorbiert

3. Zusammensetzung nach Anspruch 1, wobei die Sublimation durch Gefriertrocknung des absorbierenden Keratin-Materials erreicht wird.

4. Zusammensetzung nach Anspruch 1, wobei das Haar-Filament ein menschliches Haar-Filament ist.

5. Zusammensetzung nach Anspruch 1, wobei das Haar-Filament tierisches Haar- oder Woll-Filament ist.

6. Zusammensetzung, die durch ein Verfahren hergestellt wird, das aufweist: Oxidieren von Haar oder Wolle, um einen wesentlichen Anteil der Zystin-Disulfid-Bindungen im Haar oder der Wolle zu spalten; Entfernen im Wesentlichen aller Proteine und Peptide, die bei neutralem pH-Wert wasserlöslich sind, von der Zusammensetzung; Aufquellen der Zusammensetzung durch Sättigen mit einer wässrigen Lösung; und Gefriertrocknen der Zusammensetzung.

7. Verwendung einer Zusammensetzung nach einem der vorstehenden Ansprüche bei der Bereitstellung eines Wasser-absorbierenden Films.

8. Verwendung einer Zusammensetzung nach einem der Ansprüche 1 bis 6 bei der Bereitstellung einer Wundauflage, eines Gerüstmaterials für das Zellwachstum, eines Materials zur kontrollierten oder fortwährenden gezielten Medikamentenabgabe, eines Hautpflege-Produkts, eines Wasserabsorbierenden Verbandsmaterials, einer Windel, eines absorbierenden Frauenhygiene-Produkts, oder eines Stoffs zur Absorption von Umgebungs-Fluiden.

## Revendications

1. Composition renfermant un matériau absorbant à base de kératine obtenue à partir d'un filament capillaire, ce matériau absorbant étant essentiellement exempt de protéines et de peptides de kératine solubles dans l'eau, et le filament étant obtenu par saturation avec un solvant aqueux pour obtenir un filament expansé puis par élimination de l'eau par sublimation pour obtenir un matériau absorbant.

2. Composition conforme à la revendication 1, dans laquelle le matériau absorbant absorbe au moins vingt fois son poids d'eau.

3. Composition conforme à la revendication 1, dans laquelle la sublimation est effectuée par séchage par congélation du matériau absorbant à base de kératine.

4. Composition conforme à la revendication 1, dans laquelle le filament capillaire est un filament capillaire humain.

5. Composition conforme à la revendication 1, dans laquelle le filament capillaire est un filament capillaire animal ou un filament de laine.

6. Composition obtenue par la mise en oeuvre d'un procédé comprenant les étapes consistant à : oxyder des cheveux ou de la laine pour permettre de rompre la plus grande partie des liaisons disulfure de la cystine dans les cheveux ou la laine, éliminer essentiellement de la composition, toutes les protéines et tous les peptides qui sont solubles dans l'eau à pH neutre faire gonfler la composition par saturation avec une solution aqueuse et sécher la composition par congélation.

7. Utilisation d'une composition conforme à l'une quelconque des revendications précédentes pour la préparation d'un film absorbant l'eau.

8. Utilisation d'une composition conforme à l'une quelconque des revendications 1 à 6 pour la préparation d'un pansement, d'un support de croissance cellulaire, d'un matériau permettant de délivrer un médicament de manière contrôlée ou soutenue, d'un produit de soin cutané, d'un pansement absorbant l'humidité, d'une couche, d'un produit d'hygiène féminine ou d'un produit absorbant des liquides de l'environnement.
